# EUROPEAN PATENT APPLICATION

(11) **EP 2 939 995 A1**
(43) Date of publication of application: **04.11.2015**
(21) Application number: 14166757.6
(22) Date of filing: 30.04.2014
(51) Int. Cl.: C07C 29/04, C07C 31/10

(54) **Improved water management for the production of isopropyl alcohol by gas phase propylene hydration**

(71) Applicant: Sasol Solvents Germany GmbH, 20537 Hamburg (DE); Technische Universität Clausthal, 38678 Clausthal-Zellerfeld (DE)
(72) Inventor: The designation of the inventor has not yet been filed
(74) Representative: Smith, Julian Philip Howard

(57) **Abstract**

The present invention provides an improved water management for the isopropyl alcohol production from propylene and water in the gas phase catalysed by an acidic catalyst. The invention improves the efficiency of the water separation and the overall reactor performance.

## Description

The present invention is concerned with an improved water management for the production of isopropyl alcohol (IPA) from propylene and water in the gas phase catalysed by a supported acidic catalyst. The improvement lies in an increase in the efficiency of the water separation and the overall reactor performance.

### Description of the prior art

Four different types of processes for the synthesis of isopropyl alcohol from propylene are operated worldwide on a commercial scale. It is common to all of that processes that isopropyl alcohol is obtained in an aqueous solution with an isopropyl alcohol content of 10 to 15 % by weight. The processes use as a first separation step, a pre-concentration column, in order to separate an isopropyl alcohol/water azeotrope from excess water. The pre-concentration column has a considerable energy demand. The top product is then purified by heteroazeotropic distillation.
1. In the weak acid process, described for example in US 4471142 A, propylene reacts with sulphuric acid to isopropyl sulphate which is hydrolyzed with an excess of water forming isopropyl alcohol. The raw product is a diluted aqueous solution.
2. The ion exchange resin process, disclosed e.g. in EP 0257511 B1, US 3994983 A and US 3996298 requires an excess of water versus the propylene feed to ensure high selectivity. Higher olefin feed rates lead to unselective methyl pentene production. Again, isopropyl alcohol is obtained as an aqueous solution.
3. In the heteropoly-acid process, disclosed e.g. in US 3996298 A, a water-soluble silicotungstic acid catalyst is used. Crude isopropyl alcohol is discharged in an aqueous solution also containing the catalyst. Isopropyl alcohol is separated by a pre-concentration column prior to azeotropic distillation and the aqueous catalyst solution is recycled.

All of the three processes need a high ratio of water to propylene in the reactor section (synthesis step) to operate efficiently and are operated in a liquid aqueous phase.
4. In contrast, the fourth process, as taught e.g. in DE 2034963 A1, US 6881700 B2 or US 5208195 A, is a gas phase process catalyzed by phosphoric acid. This is the only process which is operated with a propylene excess relative to water in the reactor section. The isopropyl alcohol leaves the reactor with the propylene gas stream which is then washed with water obtaining an aqueous diluted isopropyl alcohol solution similar to the above mentioned liquid phase processes.

All four processes require recovering isopropyl alcohol from a diluted aqueous phase with the need for several distillation units, see for example US 5986148 A, US 5208387 A and US 3996298 A.

### Object of the invention

The object of the invention is to improve the recovery of isopropyl alcohol in the phosphoric acid catalyzed process. The excess use of water for washing an isopropyl alcohol containing gaseous reactor effluent, should be avoided in order to reduce the purification efforts.

### Summary of the invention

The process according to the invention relates to the conversion of propylene with water to isopropyl alcohol comprising the steps of
a) reacting in a reactor, preferably in a tube reactor, propylene with water both being in the gas phase to produce a gaseous reaction stream comprising propylene, water and isopropyl alcohol,
b) condensing at least part of the gaseous reaction stream to obtain a condensate comprising at least water, isopropyl alcohol and propylene in a decanter,
c) separating the condensate by decantation in the decanter in at least two separate liquid phases, one being an organic phase comprising by mole predominantly propylene and isopropyl alcohol and the other one being an aqueous phase comprising by mole predominantly water,
   wherein
   - the condensate comprises an aqueous and an organic phase containing i) water and ii) propylene and IPA in a molar ratio of 1 mole a) to greater than 1 mole b) (propylene plus IPA), preferably of 1 mole i) to greater than 2 moles ii),
   - the condensate comprises no more than 35 mol% isopropyl alcohol,
   - the reactor is operated to obtain a (maximum) temperature of the reactant(s) between 170°C and 220°C at least at one point within the reactor (typically reached at the exit of the reactor if the temperature is not uniform within the reactor) and a pressure of at least 35 bar, preferably between 35 bar and 45 bar and
   - the decanter is operated at a temperature between 30 °C and 150°C, preferably between 50°C and 150°C and a pressure between 5 bar and 40 bar in order to obtain condensation.

In a preferred embodiment of the process according to the invention at least part of the water from the aqueous phase is recycled into the reactor, preferably the aqueous phase from the decanter.

The condensate may be provided by cooling and the cooling is preferably achieved by subjecting the gaseous reaction stream to throttling and/or heat exchange, wherein the throttling may in a preferred embodiment be accompanied by a pressure drop of 5 to 30 bar. The pressure and the temperature in the decanter is preferably at least 5 bars, most preferably at least 10 bars, and at least 50 °C, most preferably at least 100°C, lower than in the reactor.

The catalyst used is an acidic catalyst, preferably a phosphoric acid and most preferably on a solid support.

### Detailed description of the invention

State of the art in isopropyl alcohol production is the use of considerable amounts of water in the reaction section and/or in the separation units. The purification of dilute aqueous alcohol solutions is, generally, an expensive task due to high equipment and operating costs. Until now, there is no process disclosed which allows to avoid excessive amounts of water. The reason for this is that in conventional processes there is no organic phase present which could be a feedstock for isopropyl alcohol recovery.

For a liquid phase isopropyl alcohol production process the recovery of isopropyl alcohol from an organic phase was recently disclosed in EP 2208719 A1. A catalyst arrangement in a structured packing inside the reactor allows an organic phase which contains most of the formed isopropyl alcohol and can be withdrawn easily as reactor effluent. The purification of isopropyl alcohol is achieved by distillation of the organic phase. The catalyst arrangement disclosed in above application is also applicable for use in the reactor of the present invention.

For the production of secondary butyl alcohol by ion exchange resin catalyzed hydration of butylenes in the liquid phase the recovery from an organic phase is state of the art since many decades, see for example US 4476333. This concept is not applicable to isopropyl alcohol production due to unavoidable side reactions.

It is the object of the present invention to generate isopropyl alcohol as part of a liquid organic phase obtained from condensation of the gaseous reactor effluent of the acid catalysed reaction process, in particular phosphoric acid catalysed. The advantageous water management of the present invention ensures an efficient product separation and improved reactor operation.

The separation of isopropyl alcohol is provided from an organic phase comprising mainly propylene. The improvement is based on avoiding excessive amounts of water as they were previously used for isopropyl alcohol absorption from the gaseous reactor effluent. Instead of the absorption of isopropyl alcohol in water the separation of the isopropyl alcohol is achieved by the combination of liquefaction by condensation, decantation of the organic phase and distillation und/or stripping of the organic phase. Besides the improved water management the heat recovery used saves energy. In a first step, the reactor feed is preheated by the gaseous effluent stream. In a second step, heat is preferably transferred from the gaseous reactor effluent to the organic feed stream for stripping and/or distillation. The condensation to form the desired organic phase is supported by the Joule-Thompson effect. Further treatment of the bottom product stream of the stripper / distillation column for purifying isopropyl alcohol is state of the art and needs no further characterization.

Use of excess water in the gas phase reactor can further be avoided by a) providing multiple catalyst beds in one or more reactors and/or b) by a reduction of the water intake into the reactor via the regular feed by using side injections of water into the respective catalyst beds. This leads to a higher propylene concentration inside the reactor increasing the space time yield for the isopropyl alcohol formation. Above process steps a), b) or a) and b) lower the water amount needed which must be handled in the separation section.

If water is introduced into the reactor at least at two different positions along the reactor path the reactor preferably comprises multiple catalyst beds and the positions, where water is injected, are located between different catalyst beds.

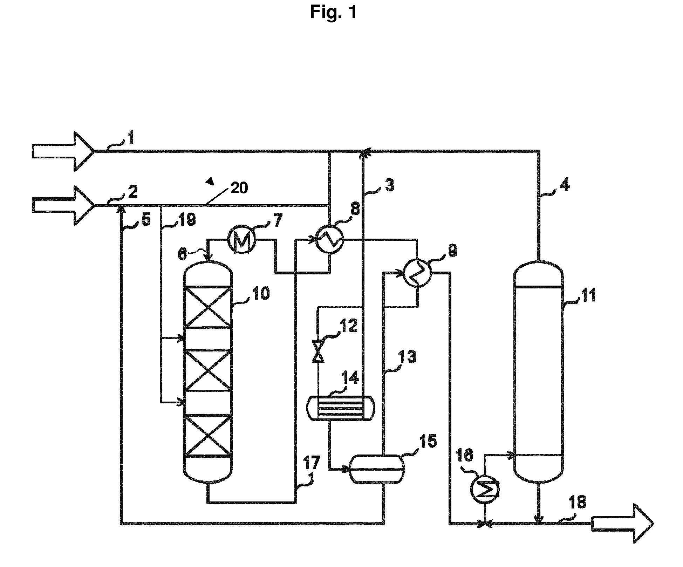

In Fig. 1 the process according to the invention is depicted.

The reactants are provided as a propylene feed stream (1) and a water feed stream (2). The water feed stream (2), which may be complemented by an aqueous recycle stream (5) from the decanter (15), is split into stream (19) and stream (20) of which (optional) stream (19) is used to inject water into intersectional gaps between the multiple catalyst beds in the reactor (10). The other part of the water feed stream (20) is merged with the propylene feed stream (1) which is complemented by at least one propylene recycle stream (3) from the condenser (14) and/or one stream (4) from the stripping/distillation unit (4) and is then heated with the reactor effluent stream (17) in heat exchanger (8). The adjacent heat exchanger (7) ensures complete evaporation of the feed stream (6) of the reactor feed mixture prior to entering the reactor (10). The gaseous reactor effluent stream (17) is preferably first cooled in the heat exchanger (8) and more preferably further cooled in a second cooling step in the heat exchanger (9) by heating the intermediate product stream (13) from the decanter (15). The pressure of the cooled gas stream is preferably reduced by valve (12) in order to enhance the adjacent condensation in the condenser (14) by the Joule-Thompson-effect. The condensate flows to the decanter (15) while the residual gas in the condenser (14) is recycled as stream (3). The condensate splits into two liquids, an organic phase of low density and a more dense aqueous phase which is withdrawn from the decanter (15) as the aqueous recycle stream (5).

The required extend of condensation depends on the content of isopropyl alcohol to ensure a heterogeneous condensate, i.e. a liquid which splits into two immiscible phases. The organic phase containing the majority of the product isopropyl alcohol is passed as the intermediate product stream (13) through the heat exchanger (9) for heat exchange with the gaseous reactor effluent stream (17) in order to enter thereafter into the separation unit. The preheated intermediate product stream (13) is evaporated by the reboiler (16) which is the bottom product reboiler of the separation column (11), which can be a stripping or a distillation column. The column separates the intermediate product stream (13) into propylene and heavier boiling components. The top product propylene is recycled to the reactor as stream (4). State of the art is to prevent accumulation of by-products or inerts by purging of the recycle streams. Finally, the bottom product is withdrawn by stream (18) and is processed by further separation steps which are state of the art.

The present invention reduces the energy needed compared to processes as described e.g. in Patent DE 2034963 A1 where washing with huge amounts of water is required. The recovery of IPA from the condensed liquid propylene phase via distillation is favourable compared to the procedure described in DE 2034963 A1. In DE 2034963 A1 the propylene phase was not condensed but was washed with huge amounts of additional water to recover IPA from unconverted propylene. To gain pure IPA this high amount of water phase with low IPA concentration must be distilled. This leads to high energy consumption.

It depends on the desired purity of the product IPA if a stripping or a distillation column is preferred as separation column (11). In Figure 1 a stripping column is depicted, but for those who are skilled in the art it is obvious to replace this unit by a distillation column equipped with condenser, reboiler and reflux devices. Independent of which kind of separation is used, further purification is necessary and it is state of the art to separate IPA from DIPE and other by-products.

### Experimental part

The process described above was carried out in a unit comprising a reactor (10), a throttling valve (12), a condenser (14), a decanter (15) and a separation column (11).

The propylene hydration reactor was fed with inlet stream (6), including propylene stream (1) and water stream (2) and the recycle streams (3), (4) and (5) with the values given in the next tables. For the experiments a laboratory gas phase hydration reactor was filled with a phosphoric acid catalyst volume of 650 mL of hydration catalyst as described in patent US 20040029718. The reaction temperature was adjusted to 190°C at the reactor inlet, leading to a reactor exit temperature of 207°C, at a pressure of 40 bar.

**Table I**

| | stream (6) reactor inlet [g/hr] | stream (17) reactor outlet [g/hr] | stream (2) [g/hr] | stream (1) [g/hr] |
|---|---|---|---|---|
| propylene | 1664.4 | 1544 | 0 | 122.6 |
| Water | 271.4 | 227 | 45.8 | 0 |
| IPA | 11.0 | 135 | 0 | 0 |
| DIPE | 11.2 | 52 | 0 | 0 |

The reactor outlet stream (17) was fed to condenser (14) and decanter (15). The condensation in condenser (14) was supported by throttling valve (12), using the Joule-Thompson-effect. The pressure in the condenser was adjusted to 19 bar and the temperature to 50°C.

In table II the streams in the condenser (14) and the decanter (15) as measured are given (IPA = Isopropyl alcohol, DIPE = Diisopropyl ether).

**Table II**

| [g/hr] | stream (17) | stream (3) | stream (13) | stream (5) |
|---|---|---|---|---|
| Propylene | 1544 | 43.8 | 1500 | 0.1 |
| Water | 227 | 0.1 | 3.5 | 223.3 |
| IPA | 135 | 0.1 | 124.7 | 10.7 |
| DIPE | 52 | 0.1 | 45.8 | 5.9 |
| Sum | 1958 | 44.1 | 1674 | 240 |

Thereafter the organic phase (stream (13) of decanter (15)) was fed to separation column (11). The water phase from the decanter (15) was recycled by stream (5) to the reactor inlet. The separation column had an inner diameter of 50 mm and was equipped with a structured packing to improve the separation (Sulzer DX packing). In the next table the separation column (11) streams for the distillation are given.

**Table III**

| [g/hr] | stream (13) | stream (18) | stream (4) |
|---|---|---|---|
| propylene | 1500 | 2.1 | 1497.9 |
| water | 3.5 | 1.3 | 2.2 |
| IPA | 124.7 | 124.5 | 0.2 |
| DIPE | 45.8 | 40.6 | 5.2 |
| Sum | 1674 | 168.5 | 1505.5 |

To improve the heat recovery heat exchangers (8) and (9) were applied. Side stream (19) provides homogeneous water distribution in the reactor, enabling favourable temperature profiles and ensuring long catalyst lifetime. This feature was not used for the present example, but is favourable for larger reactor diameters as used in commercial plants.

## Claims

1. A process for the conversion of propylene with water to produce isopropyl alcohol comprising the steps of
- reacting in a reactor propylene with water in a gas phase to produce a gaseous reaction stream comprising propylene, water and isopropyl alcohol,
- condensing at least part of the gaseous reaction stream to obtain a condensate comprising at least water, isopropyl alcohol and propylene in a decanter,
- separating the condensate by decantation in the decanter in at least two separate liquid phases, one being an organic phase comprising by mole predominantly propylene and isopropyl alcohol and the other one being an aqueous phase comprising by mole predominantly water,
wherein
- the condensate comprises an aqueous and an organic phase, the condensate having a molar ratio of 1 mole water to greater than 1 mole of propylene and isopropyl alcohol,
- the condensate in the decanter comprises no more than 35 mol% isopropyl alcohol,
- the reactor is operated at a temperature between 170 °C and 220 °C and a pressure of at least 35 bar, preferably between 35 bar and 45 bar and
- the decanter is operated at a temperature between 30°C and 150°C and a pressure between 5 bar and 40 bar in order to obtain condensation.

2. The process according to claim 1, wherein at least part of the water from the aqueous phase is recycled into the reactor, preferably the aqueous phase from the decanter.

3. A process according to claim 1 or 2, wherein water is introduced into the reactor additionally at least at two different positions along the reactor path.

4. A process according to claim 3, wherein the reactor comprises multiple catalyst beds and the positions are located between different catalyst beds.

5. A process according to one or more of the proceeding claims, wherein the condensation is provided by cooling and the cooling is preferably achieved by subjecting the gaseous reaction stream to throttling and/or heat exchange.

6. A process according to claim 5, wherein the throttling is accompanied by a pressure drop of 5 to 30 bar.

7. The process according to one or more of the proceeding claims, wherein the reaction is catalysed by phosphoric acid preferably on a solid support.

8. The process according to one or more of the proceeding claims, wherein at least a part of the organic phase from the decanter is subjected to a stripper to recover unconverted propylene and for recycling the unconverted propylene into the reactor.

9. The process according to one or more of the proceeding claims, wherein the condensate comprises i) water and ii) propylene and isopropyl alcohol in a molar ratio of 1 mole i) to greater than 2 moles ii).

10. The process according to one or more of the proceeding claims, wherein the pressure and the temperature in the decanter is at least 5 bars, preferably at least 10 bars and at least 50 °C, and most preferably at least 10 bars and at least 100 °C, lower than in the reactor.
